# EUROPEAN PATENT APPLICATION

(11) **EP 1 837 041 A1**
(43) Date of publication of application: **26.09.2007**
(21) Application number: 06270031.5
(22) Date of filing: 20.03.2006
(51) Int. Cl.: A61L 31/04, A61L 31/10

(54) **Tissue-adhesive materials**

(71) Applicant: Tissuemed Limited, Leeds LS14 6UF (GB)
(72) Inventor: Kettlewell, Graeme, LS14 6UF, Leeds (GB); Mandley, David, LS14 6UF, Leeds (GB); Fortune, David, LS14 6UF, Leeds (GB); Thompson, Ian, LS14 6UF, Leeds (GB); Morris, Diane, LS14 6UF, Leeds (GB)
(74) Representative: Jones, Stephen Anthony

(57) **Abstract**

A multi-lamellar tissue-adhesive sheet has a first layer comprising a film-forming polymer, to which first layer is bonded a second layer of material containing tissue-reactive functional groups. The film-forming polymer is preferably a basic polysaccharide, particularly chitosan or a derivative thereof. The tissue-reactive functional groups are preferably NHS ester groups. The sheet is useful for joining a tissue surface to another tissue, or for sealing a tissue surface.

## Description

### Field of the Invention

This invention relates to a flexible sheet suitable for use as a tissue adhesive and sealant, and intended for topical application to internal and external surfaces of the body, for therapeutic purposes. The invention also relates to a process for the preparation of such a sheet, and to methods of using such a sheet. In particular the invention relates to a self-adhesive, biocompatible and hydratable polymeric sheet, which may be used for therapeutic purposes such as wound healing, joining, sealing and reinforcing weakened tissue, and for delivery of therapeutic agents, and to a process for preparing, and methods of using, such a sheet. The invention further relates to implantable medical devices coated with similar material to that of the sheet.

### Background of the Invention

There is considerable interest in the use, for a number of surgical or other therapeutic applications, of materials that adhere to biological tissues, eg as an alternative to the use of mechanical fasteners such as sutures, staples etc. Formulations of such materials that have hitherto been proposed include viscous solutions or gels that are either manufactured in that form or are prepared immediately prior to use by mixing of the ingredients. Such formulations are then applied to the tissue surface using a suitable applicator device such as a syringe.

Formulations of the type described above suffer from a number of disadvantages. If the formulation is of low viscosity, it may spread from the area of application and hence be difficult to apply precisely to the desired area of tissue. If the formulation is more viscous, on the other hand, it may be difficult to dispense. In either case, the formulation, being prepared in hydrated form, may have a limited lifetime and may be subject to premature curing. It may therefore be necessary for the whole of the formulation to be used at once or discarded. Also, the preparation of formulations immediately prior to use by mixing of ingredients is obviously laborious and time-consuming, and may require the use of additional apparatus. In addition to these drawbacks, the degree of adhesion between tissue surfaces that is provided by such formulations may be less than would be desired.

Formulations of tissue adhesive materials have also been applied to a suitable support for application to the tissue surface. The use of therapeutic materials in the form of a sheet, patch or film, for topical administration to either internal or external organs of the body, is well documented for a wide range of medical applications. A disadvantage of products proposed hitherto, however, is that the degree of adhesion to the underlying tissue, particularly in the longer term, may be inadequate. While the initial adhesion may be satisfactory, the sheet may subsequently become detached from the tissue, often after only a few seconds or minutes, eg as a result of hydration of the sheet following its application. In addition, the flexibility of the product may be insufficient for it to conform readily to the surface to which it is applied, which may also have an adverse effect on its adhesion.

As a result of the inadequate adhesion of these products, it may be necessary to provide further reinforcement, eg through mechanical attachment using sutures, staples or the like. Alternatively, energy (eg light or heat energy) may be applied in order to initiate chemical bonding of the adhesive formulation to the underlying tissue, and hence bonding of the tissue surfaces to each other. Clearly, such approaches introduce further drawbacks. The use of mechanical fastenings such as sutures or staples is often the very thing that the use of such products is intended to replace or avoid. In many instances, the use of such fastenings is either not wholly effective (eg on the lung) or undesirable, as their introduction gives rise to further areas of tissue weakness. The use of external energy requires the provision and operation of a source of such energy. Such energy sources may be expensive and difficult to operate, particularly in the confines of an operating theatre or similar environment. Also, the use of external energy for attachment can be both time-consuming and (in some cases) requires significant careful judgement on the part of the surgeon, to evaluate when sufficient energy has been delivered to effect attachment without damaging the underlying tissue.

A disadvantage of sheet-type products for applications as described above is that they may lack the degree of flexibility that may be necessary or desirable for many applications. This is particularly so for products used in the increasingly important field of endoscopic (keyhole) surgery, which may require the product to be folded or rolled into a compact configuration prior to introduction into the body. Attempts to render such products more flexible, eg by the inclusion of plasticisers, may have the effect of reducing the adhesiveness of the product.

There have now been devised improvements to tissue-adhesive sheets or the like of the general type described above, and to related applications of tissue-adhesive material, that overcome or substantially mitigate the above-mentioned and/or other disadvantages of the prior art.

### Brief Summary of the Invention

According to a first aspect of the invention, there is provided a multi-lamellar tissue-adhesive sheet having a first layer comprising a film-forming polymer, to which first layer is bonded a second layer of material containing tissue-reactive functional groups.

The sheet according to the invention is advantageous primarily in that it bonds effectively to tissue, enabling it to be used in a variety of medical applications. The sheet has been found to offer improved flexibility and yet to retain good adhesiveness. In preferred embodiments, the sheet exhibits good initial adhesion to the tissue to which it is applied (and may thus be described as "self-adhesive"), and furthermore remains well-adhered to the tissue over a longer timescale. Without wishing to be bound by any theory, it is believed that the initial adhesion of the sheet to the tissue is attributable to electronic bonding of the sheet to the tissue, and this is supplemented or replaced by chemical bonding between the tissue-reactive functional groups of the formulation and the tissue, in particular between amine and/or thiol groups on the tissue surface and the tissue-reactive functional groups of the sheet.

Initial adhesion of the sheet to the tissue surface is believed to be due to Van der Waals forces and/or hydrogen bonding between the sheet and the tissue surface. On contact with the tissue surface the sheet becomes hydrated, thereby causing reaction between the tissue-reactive functional groups and the underlying tissue surface. Such reactions between the tissue-reactive functional groups and the underlying tissue result in high adhesion between the sheet and the tissue surface. The sheet may absorb physiological fluids (as a consequence of application onto exuding tissue surfaces), and any additional solutions used to hydrate the sheet following application (such fluids can be commonly used solutions used in surgical irrigation), becoming more compliant and adherent to the tissue surfaces, and thereby providing an adhesive sealant, haemostatic and pneumostatic function.

The use of the sheet reduces or eliminates the need for additional means of mechanical attachment to the tissue (eg sutures or staples), or the need to provide external energy in the form of heat or light to bring about adherence of the sheet to the underlying tissue. Another advantage of the sheet according to the invention is that it is applied to the tissue as a preformed article, rather than being prepared by mixing of materials immediately prior to use.

In addition, because the sheet is, until hydrated upon and following contact with the tissue surface, essentially inactive, the sheet is not prone to premature reaction and as a result its shelf-life may be considerable, eg more than six months when stored appropriately at room temperature.

By the term "sheet" is meant an article with a thickness that is considerably less than its other dimensions. Such an article may alternatively be described as a patch or a film.

In a second aspect of the invention, there is provided a device suitable for implantation in the human or animal body, which device carries on at least part of the external surface thereof a coating comprising a film-forming polymer, at least part of said coating being bonded to a layer of material comprising tissue-reactive functional groups.

In this embodiment of the invention, the coating of film-forming polymer provides a means for attachment to the device of the material comprising tissue-reactive functional groups, the latter material providing a means for anchoring the device in its desired position within the body. This aspect of the invention may therefore be of particular utility in relation to implantable devices that would otherwise be difficult to fix in position within the body, for instance because they are made of a material that is chemically inert and not amenable to reaction with the surrounding tissue or with chemical linking groups.

In the following detailed description of the invention, reference is made primarily to embodiments of the invention that have the form of sheets. It will be appreciated, however, that analogous comments apply, where appropriate, to embodiments of the invention involving coatings on implantable devices.

In another aspect, the invention also provides a method of joining a tissue surface to another tissue, or of sealing a tissue surface, which method comprises applying to the tissue surface a sheet according to the first aspect of the invention.

The sheet according to the invention may also be used for the delivery of one or more therapeutically active agents to the site to which the sheet is applied. In such a case, the active agent(s) may be incorporated into the sheet, eg by admixture with the other ingredients that are used in the manufacture of the sheet. Alternatively, the active agent(s) may be covalently bound to a component of the sheet. However, in other embodiments, the sheet is free of therapeutically active agents. In a similar manner, one or more therapeutically active agents may be incorporated into the material applied to the external surface of an implantable device according to the second aspect of the invention.

### Detailed Description of the Invention

### Abbreviations

- AAc: acrylic acid
- AIBN: azo-iso-butyronitrile
- DCC: dicyclohexylcarbodiimide
- DCM: dichloromethane
- DCU: dicyclohexylurea
- DMF: dimethylformamide
- DMSO: dimethyl sulfoxide
- DPBS: Dulbecco's Phosphate-Buffered Saline
- ENT: ear, nose and throat
- HPC: hydroxypropylcellulose
- *M*ₙ: number average molecular weight
- *M*_{w}: weight average molecular weight
- NHS: N-hydroxysuccinimide
- PEG: polyethylene glycol
- PTFE: polytetrafluoroethylene
- PLG: poly(DL-lactide-co-glycolide)
- poly(VP-AAc): copolymer of vinyl pyrrolidone and acrylic acid
- poly(VP-AAc(NHS): copolymer of vinyl pyrrolidone and acrylic acid NHS ester
- poly(VP-AAc-AAc(NHS)): terpolymer of vinyl pyrrolidone, acrylic acid and acrylic acid NHS ester

### Nature of the first layer

The sheet according to the first aspect of the invention has a first layer that comprises at least one film-forming polymer. The first layer may consist entirely or substantially entirely of film-forming polymer. In other embodiments, the first layer consists large of film-forming polymer. For instance, the first layer may comprise more than 80%, more than 90% or more than 95% w/w of film-forming polymer.

A variety of suitable film-forming polymers may be used to form the first layer, provided that they exhibit suitable film-forming properties together with suitability for medical applications, in particular absence of toxicity, biocompatibility and, usually, biodegradability.

Most commonly, the first layer comprises just one film-forming polymer. Alternatively, the first layer may be formed from more than one film-forming polymer.

In general, it is strongly preferable that the film-forming polymer should include functional groups that are reactive to functional groups present in the material of the second layer, so that covalent bonds are formed between the materials of the first and second layers during manufacture of the sheet, thereby enhancing the structural integrity of the finished product. Examples of suitable functional groups include amino groups and thiol groups. Film-forming polymers including such groups, especially amino groups, are thus particularly preferred.

The film-forming polymer may be synthetic, or it may be, or may be derived from, a naturally-occurring material.

Examples of synthetic polymers that may be suitable are aminated polymers such as aminated PEGs (including those sold under the trade name JEFFAMINE) and polyallylamines.

Preferred film-forming polymers, however, are polysaccharides, and in particular basic polysaccharides. A particularly preferred film-forming polymer is chitosan, the (at least substantially) N-deacetylated derivative of chitin. Chitin is a naturally abundant mucopolysaccharide and is the supporting material of crustaceans and insects. Chitin is readily obtainable from crustacean shells discarded in the food industry, and the preparation of chitosan from chitin is well known and documented. Derivatives of chitosan may also be used.

Chitosan may offer further benefits when used as the material of the first layer. In particular, chitosan is known to swell when hydrated and this may enhance the sealant action of the sheet. In addition, chitosan may exhibit useful antibacterial and haemostatic properties. Chitosan is also believed to be susceptible to attack by lysozyme, and this may be useful in providing a ready mechanism for biodegradation of the sheet.

### Nature of the second layer

The sheet according to the first aspect of the invention has a second layer that comprises a material containing tissue-reactive functional groups. That material preferably comprises one or more polymers containing tissue-reactive functional groups.

By "tissue-reactive functional groups" is meant functional groups capable of reacting with other functional groups present in the tissue surface so as to form covalent bonds with the tissue. Tissues generally consist partly of proteins, which commonly contain thiol and primary amine moieties. Many functional groups such as imido ester, p-nitrophenyl carbonate, NHS ester, epoxide, isocyanate, acrylate, vinyl sulfone, orthopyridyl-disulfide, maleimide, aldehyde, iodoacetamide, and others, may react with thiols or primary amines, and therefore constitute "tissue-reactive functional groups". As used herein, the term NHS or NHS ester is intended to encompass not only N-hydroxysuccinimide itself, but also derivatives thereof in which the succinimidyl ring is substituted. An example of such a derivative is N-hydroxysulfosuccinimidyl and salts thereof, particularly the sodium salt, which may increase the solubility of the tissue-reactive material.

Tissue-reactive functional groups that may be of utility in the present invention are any functional groups capable of reaction (under the conditions prevalent when the formulation is applied to tissue, ie in an aqueous environment and without the application of significant amounts of heat or other external energy) with functional groups present at the surface of the tissue. The latter class of functional group includes thiol and amine groups, and tissue-reactive functional groups therefore include groups reactive to thiol and/or amine groups. Examples are:
imido ester;
p-nitrophenyl carbonate;
NHS ester;
epoxide;
isocyanate;
acrylate;
vinyl sulfone;
orthopyridyl-disulfide;
maleimide;
aldehyde; and
iodoacetamide.

NHS ester is a particularly preferred tissue-reactive functional group.

In addition to tissue-reactive functional groups, the polymer(s) that make up the material of the second layer may contain functional groups that, whilst not themselves being reactive to the tissue to which the sheet is applied, do provide good contact adhesion between the sheet and the tissue. Such functional groups are referred to herein as "non-reactive functional groups". Examples of non-reactive functional groups include hydroxyl and, particularly, carboxyl groups.

It is particularly preferred that the tissue-reactive functional groups are activated derivatives of non-reactive functional groups. In certain embodiments, all of the non-reactive functional groups may be activated to form tissue-reactive functional groups. In other embodiments, only some of the non-reactive functional groups may be activated to form tissue-reactive functional groups. In the latter case, the strength of initial contact adhesion of the sheet to the tissue to which it is applied, and the strength of the longer term adhesion brought about by covalent reaction of the tissue-reactive functional groups with functional groups in the tissue may be varied and controlled by varying the proportion of the non-reactive groups that are in activated form.

NHS ester is a particularly preferred tissue-reactive functional group, and therefore preferred tissue-reactive polymers are NHS ester-rich polymers. Particularly preferred tissue-reactive polymers are poly(VP-AAc(NHS)) and poly(VP-AAc-AAc(NHS)) terpolymer.

Sufficiency of the degree of initial adhesion of a sheet to the tissue, by the bioadhesive polymer(s), can be quantitatively determined *in vitro*, for example by performing an adhesion strength test. This test is performed by allowing the sheet to adhere to a suitable substrate (secured in a fixed position), while the sheet itself is physically attached at a separate point to the load of a tensile testing apparatus, positioned so that, prior to the test, the sheet is not under load. The load cell is moveable along an axis substantially perpendicular to that along which the substrate is positioned. The test involves movement of the load cell away from the substrate, at a constant predetermined rate, until the sheet detaches from the substrate. The output of the test is a quantitative measure of the energy of adhesion for that sheet - ie the cumulative amount of energy required to break the interaction between the sheet and the substrate to which it is adhered. A suitable cumulative energy of adhesion for the sheet according to the invention would be not less than 0.5mJ.

In certain embodiments of the invention, a preferred tissue-reactive polymer is poly(VP-AAc-AAc(NHS)) terpolymer. The carboxyl groups on poly(VP-AAc) may be converted to NHS esters by reaction with NHS in the presence of DCC (see Example 9). If the acid content of the poly(VP-AAc) is determined (in moles), the proportion of acid groups converted to tissue-reactive groups may be controlled by adding the desired mole percent of NHS.

Another tissue-reactive polymer that may be used, containing hydroxyl groups, is an activated form of HPC succinate, eg HPC succinate-NHS. In this case, some of the hydroxyl groups are activated with NHS via succinic acid linkage (see Example 11).

The properties of the tissue-adhesive sheet may be optimised by inclusion of other polymers and additives.

### Plasticizers

Although in general the sheet according to the first aspect of the invention has adequate flexibility, it may nonetheless be desirable to further improve the flexibility and/or wet-strength of the sheet by the addition of one or more plasticizers to the first layer and/or the second layer. In particular, low molecular weight species such as glycerol and low molecular weight PEG (preferably *M*_{w} = 200-1000) may be incorporated into the formulations to increase flexibility. Such materials may increase the flexibility of the sheet when added at levels of up to 30% by weight of the ingredients that make up the sheet. However, the inclusion of high levels of such materials may have a detrimental effect on the adhesive performance of the sheet.

To offset this disadvantage, plasticizers may be used that are functional materials that include tissue-reactive functional groups that may participate in tissue-adhesion. Examples of such plasticizers are , -di-NHS ester functional poly(ethylene glycol) and citric acid NHS ester.

### Buffers

The reaction between tissue-reactive functional groups on the sheet and functional groups on the surface of the tissue may vary with pH. It may therefore be preferable to buffer the tissue surface immediately prior to application or, more preferably, to include a buffer in the formulation used to prepare the sheet, in particular the second layer of the sheet. The mean work of adhesion of certain sheets according to the invention to explanted porcine liver may be improved by buffering the tissue surface with pH 10.5 phosphate/carbonate buffer.

### Cross-linking of components of the sheet during manufacture

The materials of the first layer and/or the second layer may be cross-linked during the process of manufacture. Such cross-linking may increase the physical strength of the sheet and may optimise the properties of the sheet, in particular in terms of the time required for biodegradation of the sheet after it has been applied.

Cross-linking may be brought about by various means. Most preferably, however, at least one component is included in the formulation from which the first layer and/or second layer is prepared that comprises at least two functional groups which are capable of reacting with functional groups present in the material that is to be cross-linked. This component will therefore act as a cross-linking agent. Preferably, the cross-linking agent contains at least two functional groups of the same form. Thus, the cross-linking agent is most preferably a homobifunctional or homopolyfunctional cross-linking agent. Examples of suitable cross-linking agents include derivatives of relatively low molecular weight PEG, eg PEG diacrylate.

### Physical form of the sheet

The sheet may typically have an overall thickness of from 0.01 to 1 mm, typically 0.01 to 0.5mm, and more commonly 0.02 to 0.2 mm, eg about 100µm or less.

The sheet may be produced with, or subsequently cut to, dimensions of from a few square millimetres up to several tens of square centimetres.

The second layer is bonded to one surface of the first layer. Optionally, the other surface of the first layer may carry a backing layer. Such a backing layer may be particularly useful where it is desired that the other surface of the first layer should be non-adhesive. Most preferably, the material of the backing layer is a synthetic polymer. Examples of suitable polymers include PEG, polylactide and PLG. A sheet with such a non-adhesive backing layer will adhere only to the target tissue (to which the second layer of the sheet, containing the tissue-reactive functional groups, is applied) and not to surrounding tissues (eg the pleural or peritoneal wall). The backing layer will typically have a thickness of 1-50µm. The backing layer may include a visibly-absorbing chromophore to enable identification of the non-tissue contacting surface of the sheet. An example of a suitable chromophore is methylthioninium chloride.

In order to promote adherence of the backing layer to the first layer, the backing layer may be formed with a relatively small amount of a material containing functional groups that are capable of reaction with the material of the first layer. Conveniently, the materials that make up the non-adhesive layer may include a minor proportion of a similar material to that of the second layer. As such functional groups may have the effect of conferring an undesired degree of adhesiveness on the backing layer, it may be preferable for the concentration of such material within the backing layer to be graduated, so that it is greatest at the interface between the backing layer and the first layer and zero or at a minimum at the exposed surface of the backing layer.

In certain embodiments, the backing layer (where present) is apertured. Most preferably, the backing layer is formed with a regular array of perforations. The presence of such perforations may promote passage of fluid through the sheet, and hence reduce the build-up of fluid beneath the sheet, which could otherwise lead to the sheet becoming dislodged.

### Manufacture of the sheet

Most conveniently, the sheet according to the invention may be prepared by stepwise formation of the individual layers that make up the sheet.

Where the sheet contains a backing layer as described above, the backing layer may be prepared first, eg by casting a solution of the material that makes up the backing layer in a suitable solvent, either on a suitable plate or mould or onto a suitable release paper, eg a silicone-coated release paper. The cast solution is then dried or allowed to dry, optionally under conditions of elevated temperature and/or reduced pressure.

Where the backing layer is to contain functional groups that are reactive to the material of the first layer, the backing layer may be prepared by a two step procedure in which a layer of an inert, non-reactive material (eg PLG) is first cast, and then a second casting is made, of material containing the reactive functional groups. If the second casting is made on top of the first, before the first casting is fully dried and cured, the material of the second casting will diffuse into the first, leading to the formation of a concentration gradient of the reactive functional groups.

Once the backing layer has been formed, it may be perforated. In preferred embodiments, the backing layer is formed with a regular array of apertures, for example in a square or hexagonal array. The apertures may be formed, for example, by piercing.

Preferably, the apertures are between 50µm and 2mm in diameter and adjacent apertures are formed at a centre-to-centre separation of between 100µm and 5mm. Preferably, the apertures account for between 5% and 80% of the overall surface area of the backing layer.

The first layer may then be cast onto the pre-formed backing layer. Most preferably, this is followed by drying to remove solvent, and curing to achieve the desired degree of cross-linking. Curing is most preferably promoted by application of elevated temperatures (typically up to one hour or more at temperatures of up to 60°C or higher).

Finally, the second layer may be cast onto the first layer. Once again, this is preferably is followed by drying to remove solvent, and curing to achieve the desired degree of cross-linking. Curing preferably occurs at least partially at elevated temperatures (typically in excess of ten minutes and up to one hour or more at temperatures of up to 60°C).

Once manufactured, and prior to use, the sheet according to the invention will typically have a water content of less than 10% w/w, and more commonly less than 5% w/w.

Typically, implantable devices according to the second aspect of the invention may be prepared by any convenient method of applying the coating to the device. For example, the layers of the coating may be cast on the device in an analogous manner to the way in which the materials of the first and second layers are cast onto a barrier layer of a sheet, as described above. Alternatively, the coating may be applied by dipping of a device in liquid formulations or by spraying the device with liquid formulations.

### Therapeutic applications of the sheet

The sheet according to the invention is suitable for application to both internal and external surfaces of the body, ie it may be applied topically to the exterior of the body (eg to the skin) or to internal surfaces such as surfaces of internal organs exposed during surgical procedures, including conventional and minimally invasive surgery.

The sheet according to the invention is particularly suitable for surgical applications in the following areas:
Thoracic / cardiovascular
General surgery
ENT
Urology
Oral / maxillofacial
Orthopaedic
Neurological
Gastroenterology
Ophthalmology
Gynaecology / obstetrics

Possible uses are described in more detail below.

### Wound healing

The degradable nature of the sheet means that it may support and promote wound healing during both internal and topical procedures. Once the sheet begins to degrade, fibroblasts will move in and begin to deposit components of the extracellular matrix. The sheet can therefore be used as an internal or external dressing. In addition, factors such as growth factors and cAMP that are known to promote the proliferation of skin cells may be added to the formulation to assist in the healing process. The sheet may be designed to control the transmission of moisture and infectious agents, and thus be useful particularly in the treatment of burns.

### Skin closure

The sheet may be applied topically to promote wound closure (as an alternative to sutures). This may have beneficial effects in that it may reduce scarring, and the formulation and sheet may thus be useful for cosmetic purposes during minor surgery (eg in Accident & Emergency Departments). Self-adhesive properties of the sheet may make it easy to apply quickly.

### Hernia repair

The sheet may be used to provide reinforcement in hernia repair procedures. The self-adhesive attachment overcomes the potential issues faced by conventional surgical reinforcing mesh products, which require suturing or stapling in an already weakened area. The sheet for such a procedure may be engineered to have short or long term durability, depending on the degree of tissue repair required. The sheet may also be able to withstand the application of staples.

The invention may also find application in the provision of an adhesive coating to hernia mesh devices.

### Anastomosis

The sheet provides a means for rapid sealing of, and prevention of leaks in, joined tubular structures such as blood vessels, and vascular and bladder grafts, and the GI tract. The ability of the sheet to support tissue repair may be of particular value if used in nerve repair.

### Sealing large areas of tissue

The good sealing and handling properties of the sheet, combined with its self-adhesive properties and ability to cover a large surface area, mean that it may be of particular use in sealing resected tissue surfaces - in particular those where diffuse bleeding is an issue (eg the liver). The sheet also provides an ideal support matrix for tissue repair at such sites. This could also be applicable to limiting leakage of cerebro-spinal fluid following neurological surgery.

### Sealing air leaks

In addition to the patch properties described above, the high tensile strength and good inherent elasticity of the sheet (after hydration and reaction of the tissue-reactive functional groups), make it particularly suitable for sealing air leaks in the lung, particularly following lung resection. Again, after effecting a seal, the sheet provides an ideal support matrix for tissue repair at such sites.

### Haemostasis

The sheet may be applied to a bleeding area, acting as a physical barrier.
The tissue-reactive material in the sheet may immobilise proteins and thereby promote haemostasis.

### Therapeutic agent administration

Drugs and other therapeutic agents (including biologically active agents such as growth factors, and even cells and cellular components) may be added to solution(s) used to form the components of the sheet, or covalently linked to components prior to their use in the manufacture of the sheet. Once the sheet is in place, following application to the desired site, the drug will be slowly released, either by diffusion or by engineering the sheet, eg by controlling the degree of cross-linking within that sheet, so that as it degrades over time the drug is released. The rate of release can be controlled by appropriate design of the sheet. The sheet may thus provide a means for delivering a known amount of drug either systemically or to a precise locus. The drug may be directly bound to a component of a solution used in the manufacture of the sheet, or simply dispersed in the solution.

### Prevention of post-surgical adhesions

Post-surgical adhesion, the formation of undesired connective tissue between adjacent tissues, is a serious problem which can give rise to major post-surgical complications. It is a particular problem in bowel surgery where it can cause, for instance, twisting of the bowel, which may then necessitate further surgical intervention. The application of sheet material in accordance with the invention to tissues exposed in a surgical procedure can be effective in preventing post-surgical adhesions between that tissue and neighbouring tissues.

### Minimally invasive procedures

The use of minimally invasive techniques for taking tissue samples by biopsy, inserting devices, delivery of therapeutic agents and performing surgical procedures is rapidly developing as an alternative choice to traditional "open" surgery. Minimally invasive procedures typically result in less pain, scarring, quicker recovery time and fewer post-operative complications for patients, as well as a reduction in health care costs. Procedures are undertaken using specially designed instruments which are inserted through small keyhole-sized surgical incisions. The sheet may be introduced into the body via existing and specially designed minimally invasive surgery instruments and trocar systems, and the sheet may be shaped or prepared to an appropriate size and configuration, including buttresses for use with stapling devices. The flexibility of the sheet enables it to be formed into a configuration of reduced size, for instance by folding or rolling the sheet, and hence facilitates the use of the sheet in minimally invasive surgery procedures and/or other procedures where access is restricted. The good initial contact adhesion of the sheet to the tissue to which it is applied may also be particularly useful in such procedures.

### Brief Description of Drawing

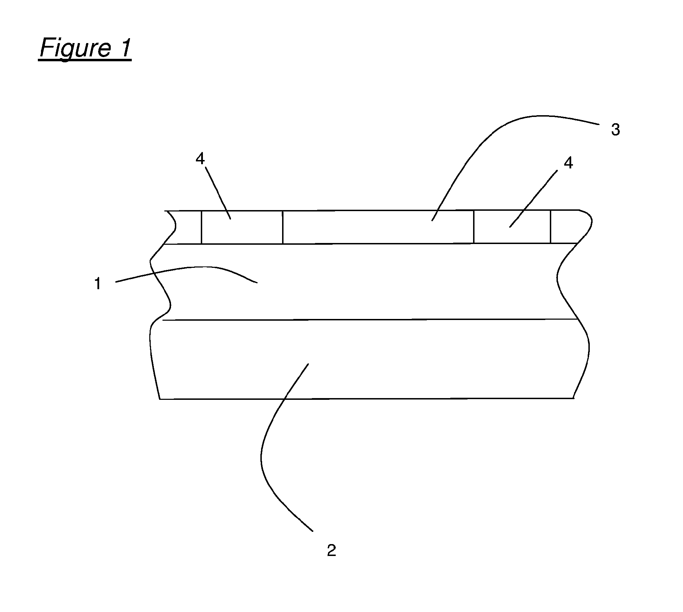

Figure 1 shows schematically, and not to scale, the structure of a tissue-adhesive sheet according to the invention.

### Detailed Description of Preferred Embodiments

The invention will now be described in greater detail, by way of illustration only, with reference to the following Examples.

### Example 1

### Preparation of multi-lamellar sheet

A multi-lamellar tissue-adhesive sheet according to the invention is shown schematically in Figure 1. The sheet comprises a central first layer 1 formed of chitosan. Applied to a first surface of the first layer 1 is a second layer 2 of poly(VP-AAc-AAc(NHS)). The other surface of the first layer 1 carries a backing layer 3, with a regular array of perforations 4.

The sheet is prepared as follows:

### 1.1 Preparation of backing layer 3

In the first step, a film was cast on silicone release paper from a 8g/60ml 15/4 v/v DCM/MeOH solution of 9:1 w/w PEG:poly(VP-AAC(NHS)) using a device of the type referred to as a K-bar (supplied by RK Print, Royston, UK). This was dried overnight under reduced pressure at room temperature

The cured film was subsequently perforated using a heated perforating device in the form of a press that had been adapted for the purpose. The press is fitted with a heated plate, the underside of which is formed with a regular array of pyramidal projections.

The perforations 4 were created by applying the heated plate with pressure to the film. This softened the polymer film, which redistributed itself around the small pyramids. The heated plate is set to 90°-C and pressed for 5s. The perforation size was approximately 0.5 mm with centre separations of 2.5 mm.

### 1.2 Preparation of first layer 1

The perforated film (backing layer 3) prepared in Example 1.1 was taped to a PTFE-coated stainless steel sheet and a solution of chitosan (13g/500ml 1% acetic acid) was cast over the film using a K-bar. The solution was sufficiently viscous that it did not pass to a significant extent through the perforations in the barrier layer. The solution was then dried at 60°C at atmospheric pressure for up to one hour before being removed from the oven and allowed to cool.

### 1.3 Preparation of second laver 2

Once the first layer1 , prepared in Example 1.2, was cool, a 3g/30ml 15/4 v/v DCM/MeOH solution of poly(VP-AAc-AAc(NHS)) was cast onto the surface of the first layer 1. This was dried at room temperature for approximately 10 minutes followed by 20 minutes at 60°C at atmospheric pressure.

The resulting film was flexible, clear and approximately 150-200µm in thickness. The first layer 1 and second layer 2 are of approximately equal thickness, and the backing layer 3 was of lesser thickness.

### Example 2

### Sheet with cross-linked first layer

Sheets may be prepared as described in Example 1, save that solution that is cast to form the first layer may contain both chitosan and poly(VP-AAc(NHS)) in ratios of 4:1 to 9:1 w/w. The poly(VP-AAc(NHS)) and chitosan may be dispersed in solvent and the two solutions then mixed prior to being cast. The inclusion of poly(VP-AAc(NHS)) leads to cross-linking of the first layer, thereby increasing the cohesive strength of the finished product, particularly when hydrated during use.

### Example 3

### Sheets with second layer formed from poly(VP-AAc(NHS))

Sheets may be prepared as described in Example 1, save that poly(VP-AAc(NHS)) may be used to form the second layer, instead of poly(VP-AAc-AAc(NHS)).

### Example 4

### Sheets with cross-linked second layer

Sheets may be prepared as described in Example 1 or Example 3, but with the inclusion of up to 1.2% w/w of PEG diacrylate in the solution from which the second layer is cast. This leads to cross-linking of the second layer.
NB: This procedure differs from the use of PEG diacrylate described above, in which the cross-linking agent is used in the synthesis of the tissue-reactive polymer that is then used in the formation of the second layer.

### Example 5

### Sheets of reduced thickness

By the use of appropriate quantities of material, sheets may be produced as described in Example 1, but with overall thicknesses of less than 100µm. Typically, the thickness of the first and second layers may be of the order of 30µm and of the barrier layer 10µm.

### Example 6

### In vitro adhesive performance

The *in vitro* adhesive performance to liver of a sheet according to the invention has been quantified using a Zwick universal testing machine. After 5 minutes immersion in DPBS, the mean work of adhesion is up to l7mJ for a 15mm x 15mm sample. After 60 minutes immersion in DPBS, the mean work of adhesion is up to 11 mJ for the same sample size.

### Example 7

### In vivo adhesive performance

*In vivo* performance of a sheet according to the invention has been demonstrated on a 6mm punch biopsy to a rabbit liver. A 2cm diameter sample was applied to the wound and held in place with moderate pressure for 30 seconds. After 5 minutes, the liver was returned to the peritoneal cavity for thirty minutes. After thirty minutes, the liver was externalised and the wound sites inspected. The sheet remained in place and there was no evidence of leakage from around the edges of the film or through the film itself and furthermore, a clot had formed in the wound site.

### Example 8

### Preparation of poly(VP-AAc(NHS))

600ml of toluene is heated to 80°C in a water bath whilst bubbling oxygen free nitrogen through the solvent for 30 minutes to remove dissolved oxygen. 64.88g (0.58 moles) of N-vinyl pyrrolidone and 10.11g (0.14 moles) of acrylic acid are added to the toluene followed immediately by the addition of 0.144g (8.8 × 10-⁴ moles) of AlBN dissolved in 3ml of toluene. The reaction temperature is maintained at 80°C for 17-19 hours under a nitrogen blanket. The polymer is isolated by precipitation from 3000ml of 1:1 v/v hexane/diethyl ether followed by filtration under reduced pressure. The polymer is washed three times with 600ml of diethyl ether before being dried under vacuum at 40°C for 72 hours.

The acrylic acid content of the polymer is determined by titration against 1.0M NaOH. 50g of poly(VP-AAc) containing 0.10 moles of acrylic acid is dissolved in 400ml of DMF. 0.10 moles (11.58g) of N-hydroxysuccinimide is added to the solution and once all the solids have completely dissolved, 0.10 moles (20.74g) of DCC dissolved in 25ml of DMF is added to the reaction. The solution is stirred at 25°C for at least 96 hours before being filtered to remove a reaction by-product, dicyclohexylurea. The polymer is isolated by precipitation from 3200ml of 5:1 v/v hexane/iso-propanol and filtration under reduced pressure. The polymer is purified further by three successive washes with 425mi of diethyl ether and then dried under reduced vacuum at 40°C for 72 hours.

Residual amounts of contaminants such as solvents, unreacted monomer, DCC and DCU are removed by Soxhlet extraction using iso-propanol as the extraction solvent.

### Example 9

### Synthesis of poy(VP-AAc-AAc(NHS)) terpolymer

400ml of toluene in a 500ml round bottomed flask is heated using a thermostatted water bath set to 80°C. The toluene is deoxygenated by bubbling oxygen free nitrogen through the solvent for 30 minutes. 31.6g (0.28 moles) of N-vinyl pyrrolidone and 20.6g (0.28 moles) of acrylic acid are added to the toluene immediately followed by 0.1g (6.1 x x10⁻⁴ moles) of 2,2 -azobis(2-methylpropionitrile). The reaction is left at 80°C for 17-19 hours. The polymer is isolated by precipitation in 2000ml of 1/1 v/v hexane/diethyl ether followed by filtration under reduced pressure. The polymer is washed three times with 300ml of diethyl ether and finally dried under vacuum at 40 °C.

The acid content of the poly(VP-AAc) copolymer is determined by titration against 1.0M sodium hydroxide. 50mol% of the acid groups are converted to NHS ester by reaction with NHS in the presence of DCC. Briefly, 133.7g of poly(VP-AAc) containing 0.77 moles of acrylic acid functionalities and 44.54g (0.38 moles) of NHS are dissolved in 1 000ml of DMF at 25 °C. 79.77g (0.38moles) of DCC is dissolved in 137m1 of DMF and added to the polymer solution and the reaction is stirred at 25°C for 96 hours. The reaction by-product, dicyclohexylurea, is removed by filtration under reduced pressure using a 10-16 m sintered glass funnel. The polymer is isolated by adding to 1250ml of iso-propanol followed by precipitation from 5000ml of diethyl ether followed by filtration. The polymer is washed three times in 1000ml of diethyl ether and then dried at 40°C under reduced pressure.

The polymer may be purified further to remove trace amounts of contaminants by a number of commonly known methods, for example, Soxhlet extraction, dialysis or washing with using a suitable solvent such as iso-propanol. Furthermore, drying at elevated temperature under reduced pressure may remove trace amounts of solvents and other volatile matter.

Approximate molecular weights *M*ₙ = 2-5,000, *M*_{w} = 10-30,000.

### Example 10

### Alternative synthesis of poly(VP-AAc-AAc(NHS)) terpolymer

1500ml of DMSO in a 2000ml round bottomed flask is heated using a thermostatted water bath set to 80°C. The DMSO is deoxygenated by bubbling oxygen free nitrogen through the solvent for 30 minutes. 89.0g (0.80 moles) of N-vinyl pyrrolidone and 61.0g (0.85 moles) of acrylic acid are added to the toluene immediately followed by 0.03g (1.8x x10⁻⁴ moles) of 2,2 -azobis(2-methylpropionitrile). The reaction is left at 80°C for 17-19 hours and then allowed to cool to room temperature. 97.8g (0.85 moles) of NHS is dissolved in the polymer solution followed by 87.6g (0.43moles) of DCC dissolved in 180ml of DMF. This reaction is left stirring at room temperature for 24 hours. The reaction by-product, dicyclohexylurea, is removed by filtration under reduced pressure using a 16-40 m sintered glass funnel. The polymer is isolated by adding to 1700ml of iso-propanol followed by precipitation from 9350ml of diethyl ether followed by filtration. The polymer is washed three times in 2500ml of diethyl ether and then dried at 40°C under reduced pressure.

The polymer may be purified further to remove trace amounts of contaminants by a number of commonly known methods, for example, Soxhlet extraction, dialysis or washing with using a suitable solvent such as iso-propanol. Furthermore, drying at elevated temperature under reduced pressure may remove trace amounts of solvents and other volatile matter.

Futhermore, the polymer may be purified further, and the consistency of the solid improved by dissolving in 15/4 v/v DCM/MeOH and then precipitation from diethyl ether. This is then followed by successive washing with diethyl ether and then drying at 40°C under reduced pressure.

### Example 11

### Synthesis of HPC-succinate NHS

10g of HPC (*M*_{w} approx 370,000) is dissolved in 350ml of anhydrous N-methylpyrrolidone at 80°C in a thermostatted water bath. 1.4g (0.014 moles) of succinic anhydride is dissolved in the solution along with 1.71 g (0.014 moles) of 4-dimethylaminopyridine. The reaction is left overnight at 80°C. The solution is cooled to room temperature and 400ml of iso-propanol is added. The polymer is precipitated from 3000ml of diethyl ether, filtered and washed successively with 300ml of diethyl ether. Finally, the polymer is dried under vacuum at 40°C.

This polymer is then dissolved in DMF and reacted with NHS in the presence of DCC to form the amine- and thiol-reactive NHS ester compound.

## Claims

1. A multi-lamellar tissue-adhesive sheet having a first layer comprising a film-forming polymer, to which first layer is bonded a second layer of material containing tissue-reactive functional groups.

2. A sheet as claimed in Claim 1, wherein the first layer comprises more than 80%, more than 90% or more than 95% w/w of film-forming polymer.

3. A sheet as claimed in Claim 1 or Claim 2, wherein the film-forming polymer includes functional groups that are reactive to functional groups present in the material of the second layer.

4. A sheet as claimed in any preceding claim, wherein the film-forming polymer is a basic polysaccharide.

5. A sheet as claimed in Claim 4, wherein the film-forming polymer is chitosan or a derivative thereof.

6. A sheet as claimed in any preceding claim, wherein the tissue-reactive functional groups are NHS ester groups.

7. A sheet as claimed in Claim 6, wherein the second layer comprises a tissue-reactive polymer selected from the group consisting of poly(VP-AAc(NHS)) and poly(VP-AAc-AAc(NHS)) terpolymer.

8. A sheet as claimed in any preceding claim, which has an overall thickness of from 0.01 to 1 mm, typically 0.01 to 0.5mm, and more commonly 0.02 to 0.2 mm, eg about 100µm or less.

9. A sheet as claimed in any preceding claim, wherein the side of the first layer remote from the second layer carries a non-adhesive backing layer.

10. A method of joining a tissue surface to another tissue, or of sealing a tissue surface, which method comprises applying to the tissue surface a sheet as claimed in any one of Claims 1 to 9.
